(19) Europäisches Patentamt / European Patent Office / Office européen des brevets

(11) **EP 3 311 800 B1**

(12) **EUROPEAN PATENT SPECIFICATION**

(45) Date of publication and mention of the grant of the patent:
**11.03.2020 Bulletin 2020/11**

(51) Int Cl.:
*A61K 9/00* *(2006.01)*          *A61K 9/107* *(2006.01)*
*A61K 9/50* *(2006.01)*          *A61K 36/28* *(2006.01)*
*A61P 17/00* *(2006.01)*

(21) Application number: **17197861.2**

(22) Date of filing: **23.10.2017**

(54) **COMPOSITION COMPRISING MICROSTRUCTURES FOR THE CONTROLLED RELEASE OF OZONIZED OIL**

ZUSAMMENSETZUNG ENTHALTEND MIKROSTRUKTUREN ZUR KONTROLLIERTEN FREISETZUNG VON OZONISIERTEM ÖL

COMPOSITION COMPRENANT DES MICROSTRUCTURES POUR LA LIBÉRATION CONTRÔLÉE D'HUILE OZONISÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.10.2016 IT 201600106992**

(43) Date of publication of application:
**25.04.2018 Bulletin 2018/17**

(73) Proprietor: **Project & Communications, Ltd.
London (GB)**

(72) Inventor: **CAROCCI, Giancarlo
41016 Sevilla (ES)**

(74) Representative: **Bottero, Carlo et al
Barzanò & Zanardo Milano S.p.A.
Via Borgonuovo, 10
20121 Milano (IT)**

(56) References cited:
**WO-A2-2011/129784     US-A1- 2006 074 129**

• **ÖZYILDIZ F. ET AL: "Micro-encapsulation of ozonated red pepper seed oil with antimicrobial activity and application to nonwoven fabric", LETTERS IN APPLIED MICROBIOLOGY, vol. 56, no. 3, March 2013 (2013-03), pages 168-179, XP055359140, GB ISSN: 0266-8254, DOI: 10.1111/lam.12028**

**Description**

The present invention relates to a composition comprising microstructures for the controlled release of active agents, in particular ozonized oil.

Various systems are currently known and used for conveying and releasing specific active agents, which consist of different kinds of formulations and compositions. A further function of these systems is to guarantee the protection of the active agent from possible degradation processes that the agent may encounter.

WO 2012/038061 describes polymeric nano-capsules containing a lipid matrix. In this matrix, a microemulsion (w/o) of at least one hydrophilic active agent is present, enclosed by a polymeric coating consisting of proteins, polysaccharides, polyesters, polyacrylates, polycyanoacrylates, copolymers and/or mixtures thereof.

Active agents of particular interest are ozonized oils which have shown a high versatility of use in both the medical field and in the cosmetic field.

In general, ozonized oils are oils containing unsaturated fatty acids in the molecule, which have been subjected to the action of ozone. The ozone is added to the carbon-carbon double bonds, forming molozonides. These molecules are rapidly rearranged according to the Criegee mechanism to give trioxolanes. Ozonides are generally unstable, whereas trioxolanes are relatively stable, and decompose under the action of reducing agents or enzymes and can be isolated.

The Applicant has considered the problem of facilitating the skin or percutaneous absorption of an ozonized oil, through the development of compositions that can be applied on the skin directly or through a fabric that incorporates said compositions, so as to obtain a controlled release of the active agent without requiring frequent applications.

Furthermore, the Applicant has considered the problem of obtaining compositions containing ozonized oil which do not show cytotoxicity, not only in a short time with respect to their production, but also after preservation for a long period of time (for example a year). The Applicant has in fact found that ozonized oil is an unstable molecule, and consequently it tends to decompose with time becoming slightly cytotoxic. It is therefore necessary to have a composition that has a stabilizing effect with respect to ozonized oil, so as to reduce its decomposition over time and the consequent onset of problems of cytotoxicity.

The Applicant has found that these problems, and others that are indicated in greater detail hereunder, are solved by means of a composition comprising microstructures dispersed in an aqueous phase A), wherein said microstructures comprise an oil-in-water (o/w) microemulsion, in which an ozonized oil is present, enclosed by a coating comprising a cationic surfactant containing quaternary ammonium groups, a polysaccharide and a polysaccharide modified with quaternized ammonium groups.

A first aspect of the present invention therefore relates to a composition comprising microstructures dispersed in an aqueous phase A), wherein said microstructures comprise:

an internal phase consisting of an oil-in-water (o/w) microemulsion comprising an oily phase B) dispersed in a continuous aqueous phase, said oily phase B) comprising an ozonized oil;
a coating of said internal phase comprising a cationic surfactant containing at least one quaternary ammonium group, a polysaccharide and a polysaccharide modified with quaternized ammonium groups.

The composition of the present invention advantageously allows ozonized oil to be conveyed and released, so as to facilitate cutaneous or percutaneous absorption.

Furthermore, the composition according to the invention allows the achievement of a consistent increase in the cutaneous or percutaneous absorption of ozonized oil.

A further advantages lies in the fact that the application of the above composition can be effected either directly on the skin or indirectly, i.e. through the insertion of the composition itself in textile materials, which, when coming into contact with the user's skin, gradually release the ozonized oil.

According to another aspect, the present invention relates to a composition comprising dispersed microstructures as defined above for use in the treatment of skin lesions such as ulcers, erosions, abrasions, scars and the like.

According to a further aspect, the present invention relates to the use of a composition comprising dispersed microstructures as defined above for the cosmetic treatment of the skin.

Further characteristics and advantages of the present invention will appear from the following detailed description.

According to a preferred aspect of the invention, the ozonized oil is a vegetable ozonized oil, preferably selected from: sunflower oil, olive oil, peanut oil, argan oil, grapeseed oil, jojoba oil, soybean oil, corn oil, palm oil, cotton seed oil, rapeseed oil, coconut oil, castor oil, linseed oil, borage oil, evening primrose oil, or mixtures thereof. According to a further embodiment, the ozonized oil is a ozonized non-vegetable oil, for example, ethyl oleate.

According to a further preferred aspect, the oily phase B) also comprises a non-ozonized oil, preferably a non-ozonized vegetable oil, more preferably a non-ozonized vegetable oil selected from sunflower oil, olive oil, peanut oil, argan oil, grapeseed oil, jojoba oil, soybean oil, corn oil, palm oil, cotton seed oil, rapeseed oil, coconut oil, castor oil, linseed oil, borage oil, evening primrose oil, or mixtures thereof, more preferably sunflower oil or olive oil, even more preferably sunflower oil.

The continuous aqueous phase may further comprise a polyol, selected for example from propylene glycol, glycerin, or mixtures thereof. The continuous aqueous phase preferably comprises a quantity of polyols ranging from 5% to 30%, wherein the percentages are weight percentages with respect to the total weight of the continuous aqueous phase.

According to a preferred aspect of the invention, the above continuous aqueous phase comprises a non-ionic surfactant, preferably selected from: octyl glucoside, decyl glucoside, lauryl glucoside, octyl fructoside, dodecyl maltoside, decyl maltoside, nonoxynol, polyethyleneglycol p-(1,1,3,3-tetramethylbutyl)phenylether, polyethyleneglycol dipolyhydroxystearate, palmityl alcohol, oleyl alcohol, poloxamer, polysorbate, methyl glucose dioleate, sorbitan monostearate, sorbitan monolaurate, sorbitan monopalmitate, sorbitan olivate, polyethyleneglycol stearate, polyoxyethylene stearyl ether, polyoxyethylene lauryl ether, cocamide monoethanolamine, cocamide diethanolamine, cocamide triethanolamine, lauramide diethanolamine, lauramide monoethanolamine, cocamidopropylamine oxide, decyl betaine, dodecyl betaine, tetradecyl betaine, cocoyl betaine, cocamidopropyl betaine, cocamidopropyl hydroxysultaine, cocoyl amino ethyl hydroxyethyl-glycinate, cocoyl aminoethyl hydroxyethyl carboxy glycinate or mixtures thereof.

The continuous aqueous phase preferably comprises:

| | |
|---|---|
| water: | 50% - 90%; |
| non-ionic surfactant: | 10% - 50%; |

wherein the percentages are weight percentages with respect to the total weight of the continuous aqueous phase.

With respect to the coating of the internal phase, this comprises a cationic surfactant containing at least one quaternary ammonium group.

Said cationic surfactant preferably has the formula:

$$X^-N^+R_1R_2R_3R_4$$

wherein $R_1$ is a $C_8$-$C_{20}$ alkyl, preferably $C_{10}$-$C_{16}$, linear or branched;
$R_2$, $R_3$, $R_4$, the same as or different from each other, are $C_1$-$C_4$ alkyls, linear or branched, preferably methyls;
$X^-$ is a counterion, preferably a halide ion, more preferably chloride or bromide.

Said coating also further comprises a polysaccharide with the function of a gelling agent, different from the polysaccharide modified with ammonium quaternized groups defined hereunder, preferably selected from: cellulose, carboxymethyl cellulose, hydroxyethyl cellulose, methylcellulose, hydroxypropylethylcellulose, xanthan gum, guar gum, pectins, arabinogalactans, galactomannans, carboxymethyl galactomannans, albumin, alginates, or mixtures thereof.

With respect to the polysaccharide modified with quaternized ammonium groups, moreover, this is preferably selected from guar hydroxypropyltriammonium chloride, quaternized hydroxymethylcellulose, or mixtures thereof.

The coating preferably comprises:

| | |
|---|---|
| cationic surfactant: | 5% - 30%; |
| polysaccharide: | 10% - 40%; |
| polysaccharide modified with quaternized ammonium groups; | 40% - 80%; |

wherein the percentages are weight percentages with respect to the total weight of the coating.

According to a further preferred aspect, the aqueous phase A) comprises a glycol preferably selected from: propylene glycol, ethoxy diglycol or mixtures thereof.

The aqueous phase A) preferably comprises:

| | |
|---|---|
| water: | 60% - 90%; |
| glycol: | 10% - 40%; |

wherein the percentages are weight percentages with respect to the total weight of the aqueous phase A).

The ozonized oil is preferably present in the composition according to the present invention in a quantity ranging from 0.1% to 25% by weight, more preferably from 1% to 10% by weight, with respect to the total weight of the composition.

The oily phase B) and the continuous aqueous phase are preferably present in a weight ratio ranging from 5:95 to 40:60.

The composition according to the present invention preferably has the following composition:

| | |
|---|---|
| aqueous phase A) | 20%-70% by weight; |
| internal phase | 5%-50% by weight; |
| coating | 0.1%-40% by weight |

with respect to the total weight of the composition.

The composition according to the present invention more preferably comprises:

| | |
|---|---|
| aqueous phase A) | 30%-60% by weight; |
| internal phase | 15%-40% by weight; |
| coating | 1%-30% by weight; |

with respect to the total weight of the composition.

According to a preferred aspect, the composition of the invention is in a form suitable for topic application, such as cream, gel, lotion or spray.

The composition according to the present invention preferably may be incorporated in a textile material composed of natural or synthetic fibers, for example cotton, linen, wool, silk, rayon, polyesters, polyamides, nylon or the like. This textile material may be used for the manufacturing of garments destined for coming into direct contact with the skin or sanitary articles (for example, gauzes, plasters, bandages).

According to a preferred embodiment, the composition, object of the present invention, is in the form of a suppository for rectal administration. This embodiment of the composition according to the present invention is particularly advantageous for use in the treatment of rectal ulcers. The suppository can be formulated with suitable excipients well known in the art, in particular with glycerides solid at room temperature (for example cocoa butter) and possibly other excipients, such as adsorbents, surfactants, lubricants, antimicrobial preservatives, dyes.

A further aspect of the present invention relates to a method for the preparation of a composition as defined above, which comprises:

(i) preparing the oil-in-water microemulsion by mixing the oily phase B) with the continuous aqueous phase, said oily phase B) containing ozonized oil, preferably ozonized plant oil;
(ii) encapsulating the oil-in-water microemulsion with a coating comprising a cationic surfactant containing quaternary ammonium groups, a polysaccharide and a polysaccharide modified with quaternized ammonium groups;
(iii) dispersing the microstructures thus obtained in the aqueous phase A).

As far as step (i) is concerned, this is preferably effected by using a homogenizer into which the continuous aqueous phase and the oily phase B) are introduced. Preferably, in order to prevent the entrapment of air which would be detrimental to the product, a pressure ranging from 0.3 to 0.5 bar is applied during the preparation step of the microemulsion.

The preparation of the microemulsion is preferably carried out under heat, for example at a temperature ranging from 30°C to 80°C, more preferably from 40°C to 70°C.

As far as the encapsulation step (ii) is concerned, this is preferably carried out in the same homogenizer used for the preparation of the microemulsion, at temperatures generally lower than the temperature of step (i). Said temperature can generally range from 20°C to 60°C, more preferably from 30°C to 50°C. The coating components are preferably premixed and then added to the microemulsion contained in the homogenizer. Also in step (ii), in order to prevent the entrapment of air, it is preferable to operate at reduced pressure, for example ranging from 0.3 to 0.5 bar.

With respect to step (iii) for dispersing the microstructures in the aqueous phase A), this is preferably carried out at a temperature ranging from 15°C to 25°C. Also in step (iii), in order to prevent the entrapment of air, it is preferable to operate at reduced pressure, for example ranging from 0.3 to 0.5 bar.

A further aspect of the present invention relates to a textile material treated with the composition as defined above.

Another aspect of the present invention relates to a textile material treated with the composition as defined above for use in the treatment of skin lesions, such as ulcers, erosions, abrasions, scars and the like.

A further aspect of the present invention relates to the use of the textile material as defined above for the manufacturing of garments destined for coming into direct contact with the skin, sanitary articles.

The present invention is described for purely illustrative but non-limiting purposes, according to its preferred embodiments, but it should be understood that variations and/or modifications can be made by skilled persons in the field without leaving the relevant scope of protection, as defined by the enclosed claims.

The present invention is now further described by means of some embodiment examples as set forth hereunder.

EXAMPLE 1

The following composition of the invention was prepared, with a content of ozonized sunflower oil equal to 5% by weight with respect to the total weight of the composition:

| | |
|---|---|
| aqueous phase A) | 60% by weight; |
| internal phase | 37.5% by weight; |
| coating | 2.5% by weight; |

with respect to the total weight of the composition;
wherein the above phases had the following composition:
aqueous phase A):

| | |
|---|---|
| water: | 67% by weight; |
| propylene glycol: | 33% by weight; |

wherein the percentages are weight percentages with respect to the total weight of the aqueous phase A) ;
continuous aqueous phase:

| | |
|---|---|
| water: | 80% by weight; |
| propylene glycol: | 20% by weight; |

wherein the percentages are weight percentages with respect to the total weight of the continuous aqueous phase;
oily phase B):

| | |
|---|---|
| ozonized sunflower oil: | 67% by weight; |
| PEG-30 dipolyhydroxystearate: | 33% by weight; |

wherein the percentages are weight percentages with respect to the total weight of the oily phase B);
microemulsion coating:

| | |
|---|---|
| $C_{10}$-$C_{16}$-alkyl-trimethyl ammonium chloride: | 12% by weight; |
| hydroxyethylcellulose: | 28% by weight; |
| guar hydroxypropyltriammonium | chloride: 60% by weight; |

wherein the percentages are weight percentages with respect to the total weight of the coating.

The oily phase B) and the continuous aqueous phase were used with a weight ratio equal to 20:80.

The above composition was prepared as follows.

The components of the oily phase B) were introduced into a turbine homogenizer and a variable-speed mixer. By applying a reduced pressure equal to 0.4 bar, these components were mixed at a temperature of 70°C with a turbine speed of 2,000 revs/min and a mixer speed equal to 50 revs/min, until a complete dispersion of the components had been obtained.

The continuous aqueous phase was prepared in a fuser with a mixer, maintaining the temperature at 70°C, until the complete mixing of the components had been obtained.

The mixture contained in the fuser, kept at 45°C and at a reduced pressure equal to 0.4 bar, was then sucked and introduced into the homogenizer where the oily phase B) was present, also kept at a temperature of 45°C and a reduced pressure equal to 0.4 bar. The two phases were mixed with a turbine speed equal to 2,000 revs/min and a mixer speed equal to 50 revs/min.

The components of the microemulsion coating, previously mixed with each other, were introduced into the homogenizer, which was kept at a temperature of 40°C, at a reduced pressure equal to 0.4 bar, with a turbine speed equal to 2,000 revs/min and a mixer speed equal to 50 revs/min, until a complete dispersion of the components had been obtained.

Finally, the aqueous phase A), whose components had been previously mixed, was introduced into the homogenizer, and the mixture was kept at 20°C and a reduced pressure equal to 0.2 bar, with a turbine speed equal to 2,000 revs/min and a mixer speed equal to 50 revs/min.

EXAMPLE 2 : cytotoxicity tests

Tests were carried out to evaluate the cytotoxicity of the following compositions, in order to demonstrate the stabilizing effect exerted by the composition according to the present invention with respect to ozonized oil, so as to reduce its decomposition over time and therefore the onset of cytotoxicity:

- composition according to the invention, obtained according to Example 1, shortly after being produced (Composition 1A) and after a year of storage at room temperature, protected from the light (Composition 1B);
- comparative composition in the form of a gel, obtained by mixing the same ozonized oil of Example 1 with a gelling agent (commercial product Carbopol™ Ultrez 21 of Lubrizol, modified crosslinked polyacrylate), in such quantities as to obtain a concentration of ozonized oil equal to 5% by weight with respect to the total weight of the gel; also in this case, the gel was subjected to cytotoxicity analysis shortly after being produced (Composition 2A) and after a year of storage at room temperature, protected from the light (Composition 2B).

The four compositions indicated above were subjected to a cytotoxicity test (MTT test).

As is known, the cytotoxicity test (MTT test) is a reduction test used for determining the level of metabolic activity in the eukaryote cells. This is one of the most widely used cytotoxicity tests, thanks to its simplicity, accuracy and reproducibility. The chemical base of the test is the reduction of MTT, a tetrazole compound [3-(4,5-dimethylthiazol-2-yl)-2,5-diphenyltetrazolium bromide]. MTT is a yellow substance which, after reduction, forms a purple-colored formazan salt.

The process mainly takes place in the cytoplasm and secondarily in the mitochondria and at the level of the plasmatic membrane. The activity of the reductase that catalyze the reaction is highly dependent on the concentration of the intracellular NADH and NADPH, whose levels are associated with the availability of extracellular glucose. The mitochondrial succinate dehydrogenase and the cytochrome C also take part in the reduction of MTT. Consequently, any substance or treatment that interferes with these enzymes or with the glycolysis would influence the reduction of MTT and would therefore alter the results of the cell count. As a result of these metabolic reduction processes, dark purple crystals of formazan are formed within a few hours. These crystals can be solubilized in various organic solvents, mainly alcohols. An increase or a reduction in the number of cells causes an analogous variation in the quantity of formazan formed, thus giving an indication of the degree of cytotoxicity of the product tested.

The MTT test was carried out on cultures of fibroblast L929 treated with the extract (transfer liquid as such obtained after overnight incubation of the composition under examination in the culture medium) and subsequent dilutions 1:2. The same procedure was also applied for the negative reference (internal standard), whereas the positive control (SDS) was tested at concentrations ranging from 0.00313 to 0.4 mg/ml. Sodium dodecyl sulfate (SDS), a substance with known cytotoxic effects was used as positive control, whereas a non-cytotoxic internal standard was used as negative reference. The analysis was carried out according to the ISO standard for the biological evaluation of medical devices UNI EN ISO 10993-5:2009 (E) .

More specifically, an adequate number of cells was seeded in 96-well plates. Once a semi-confluent monolayer had been reached, the cells were treated with the various concentrations of the sample tested and standards and incubated for 24 hours under standard conditions. Non-treated cells kept in the culture medium represent the negative controls. After 24 hours of contact, the plates were examined under a phase contrast microscope, the medium was carefully removed from each well, the cells were then treated with MTT (1 mg/ml) and incubated for 3 hours under standard conditions. At the end of this period, the solution of MTT was eliminated and 100 $\mu$l of isopropanol were added to each well to dissolve the formazan crystals formed. The absorbance (optical density, OD) was determined by reading the spectrophotometer at a wavelength of 540 nm.

The inhibition of the cell vitality at each tested concentration was expressed as a percentage with respect to the negative control (non-treated cells) according to the following formula:

$$\text{Inhibition \%} = 100 - [OD_X/OD_{NC}) \times 100]$$

wherein:

$OD_X$ = average optical density of the cells treated with the sample under examination at the concentration X;

$OD_{NC}$ = average optical density of the negative controls.

The values thus calculated were charted against the same concentrations. The concentration/response curves obtained allow the theoretical value of IC50 to be extrapolated, for both the samples and the standards.
The IC50 value indicates the concentration of the tested compound necessary for inhibiting the cell vitality by 50%. IC50 is a parameter which allows the cytotoxicity of a compound to be evaluated according to the following scheme:

$$IC50 \leq 0.5\,mg/ml: \quad \text{cytotoxic effect}$$
$$IC50 > 0.5\,mg/ml: \quad \text{absence of cytotoxic effect}$$

The cell vitality at each concentration tested is expressed as a percentage with respect to the negative control (non-treated cells), according to the following formula:

$$\text{Vitality \% = (OD}_X \text{ / OD}_{NC}\text{) x 100}$$

wherein $OD_X$ and $OD_{NC}$ are defined above.
The cell vitality is a parameter which allows the cytotoxic potential of a compound to be evaluated according to the following scheme:

Cell vitality < 70% : potential cytotoxic effect;
Cell vitality $\geq$ 70% : absence of cytotoxic effect.

As far as the positive control (SDS) is concerned, the cell inhibition values measured in relation to the concentration, are indicated in Table 1 (the symbol indicates absence of inhibition):

Table 1

| Concentration (mg/ml) | Cell vitality inhibition (%) |
|---|---|
| 0.4 | 96.77 |
| 0.2 | 97.48 |
| 0.1 | 97.18 |
| 0.05 | 66.55 |
| 0.025 | -- |
| 0.0125 | -- |
| 0.00625 | -- |
| 0.00313 | -- |
| IC50 = 0.046 mg/ml ||

The results of Compositions 1A, 1B, 2A, 2B, illustrated above are reported in the following tables:

Table 2

| (Composition 1A) |||
|---|---|---|
| Concentration | OD Average | Vitality (%) |
| blank | 1.021 | 100.00 |
| 100% | 0.971 | 95.10 |
| 50% | 1.168 | 114.40 |
| 25% | 0.957 | 93.73 |
| 12.50% | 0.957 | 93.73 |

(continued)

| (Composition 1A) | | |
|---|---|---|
| Concentration | OD Average | Vitality (%) |
| 6.25% | 1.052 | 103.04 |
| 3.13% | 0.888 | 86.97 |
| 1.56% | 0.979 | 95.89 |
| 0.78% | 1.026 | 100.49 |

Table 3

| (Composition 1B) | | |
|---|---|---|
| Concentration | OD average | Vitality (%) |
| blank | 1.049 | 100.00 |
| 100% | 1.031 | 98.22 |
| 50% | 1.228 | 117.72 |
| 25% | 1.017 | 110.70 |
| 12.50% | 1.017 | 96.83 |
| 6.25% | 1.112 | 106.21 |
| 3.13% | 0.948 | 89.91 |
| 1.56% | 1.039 | 99.00 |
| 0.78% | 1.086 | 103.63 |

Table 4

| (Composition 2A) | | |
|---|---|---|
| Concentration | OD average | Vitality (%) |
| blank | 1.021 | 100.00 |
| 100% | 0.992 | 97.16 |
| 50% | 1.178 | 115.38 |
| 25% | 0.997 | 97.65 |
| 12.50% | 0.957 | 93.73 |
| 6.25% | 1.053 | 103.13 |
| 3.13% | 0.988 | 96.77 |
| 1.56% | 0.979 | 95.89 |
| 0.78% | 1.026 | 100.49 |

Table 5

| (Composition 2B) | | |
|---|---|---|
| Concentration | OD average | Vitality (%) |
| blank | 1.034 | 100.00 |
| 100% | 0.650 | 62.86 |

(continued)

| (Composition 2B) | | |
|---|---|---|
| Concentration | OD average | Vitality (%) |
| 50% | 0.732 | 70.79 |
| 25% | 0.945 | 91.39 |
| 12.50% | 0.957 | 92.55 |
| 6.25% | 1.034 | 100.00 |
| 3.13% | 1.001 | 96.81 |
| 1.56% | 0.979 | 94.68 |
| 0.78% | 1.026 | 99.23 |

From the above data it can be noted that the fresh composition according to the present invention (Composition 1A) and the fresh comparative composition (Composition 2A) gave vitality values of the cells treated with the extract as such (transfer liquid at 100%) equal to 95.10% and 97.16%, respectively. Both compositions can therefore be classified as non-cytotoxic.

After a year of storage, on the contrary, the composition according to the present invention (Composition 1B) and the comparative composition (Composition 2B) gave remarkably different vitality values of the cells treated with the extract as such (transfer liquid at 100%), the first a value of 98.22%, the second a value of 62.86%. Composition 1B can therefore be classified as non-cytotoxic, whereas Composition 2B should be considered as being cytotoxic. This reveals the particular stability of the compositions according to the present invention with respect to compositions in gel form.

**Claims**

1. Composition comprising microstructures dispersed in an aqueous phase A), wherein said microstructures comprise:

   an internal phase consisting of an oil-in-water microemulsion (o/w) comprising an oily phase B) dispersed in a continuous aqueous phase, said oily phase B) comprising an ozonized oil;
   a coating of said internal phase comprising a cationic surfactant containing at least one quaternary ammonium group, a polysaccharide and a polysaccharide modified with quaternized ammonium groups.

2. Composition according to claim 1, wherein the ozonized oil is an ozonized plant oil, preferably selected from: sunflower oil, olive oil, peanut oil, argan oil, grapeseed oil, jojoba oil, soybean oil, corn oil, palm oil, cotton seed oil, rapeseed oil, coconut oil, castor oil, linseed oil, borage oil, evening primrose oil, or mixtures thereof.

3. Composition according to any one of the preceding claims, wherein the continuous aqueous phase comprises:

   water:                    50 % - 90 %;
   non-ionic surfactant:    10 % - 50 %;

   wherein the percentages are percentages by weight with respect to the total weight of the continuous aqueous phase.

4. Composition according to any one of the preceding claims, wherein said cationic surfactant contains at least one quaternary ammonium group having formula:

   $$X^-N^+R_1R_2R_3R_4$$

   wherein $R_1$ is a $C_8$-$C_{20}$ alkyl, preferably $C_{10}$-$C_{16}$, linear or branched;
   $R_2$, $R_3$, $R_4$ are, equal or different from each other, $C_1$-$C_4$ alkyls, linear or branched, preferably methyls;
   $X^-$ is a counterion, preferably halide ion, more preferably chloride or bromide.

5. Composition according to any one of the preceding claims, wherein said polysaccharide, different from the polysaccharide modified with ammonium quaternized groups, is selected from: cellulose, carboxymethylcellulose, hydroxyethyl cellulose, methylcellulose, hydroxypropylethylcellulose, xanthan gum, guar gum, pectins, arabinogalactans, galactomannans, carboxymethyl galactomannans, albumin, alginates, or mixtures thereof.

6. Composition according to any one of the preceding claims, wherein said polysaccharide modified with ammonium quaternized groups, is selected from: guar hydroxypropyltriamonium chloride, quaternized hydroxymethylcellulose, or mixture thereof.

7. Composition according to any one of the preceding claims, wherein said coating comprises:

| | |
|---|---|
| cationic surfactant: | 5 % - 30 %; |
| polysaccharide: | 10 % - 40 %; |
| polysaccharide modified with quaternized ammonium groups; | 40 % - 80 %; |

wherein the percentages are percentages by weight with respect to the total weight of the coating.

8. Composition according to any one of the preceding claims, wherein the ozonized oil is present in an amount from 0.1% to 25% by weight, preferably from 1% to 10% by weight, with respect to the total weight of the composition.

9. Composition according to any one of the preceding claims, wherein the oily phase B) and the continuous aqueous phase are present in a weight ratio from 5:95 to 40:60.

10. Composition according to any one of the preceding claims having the following composition:

| | |
|---|---|
| aqueous phase A) | 20%-70% by weight; |
| internal phase | 5%-50% by weight; |
| coating | 0.1%-40% by weight; |

with respect to the total weight of the composition.

11. Method for preparing a composition according to any one of the preceding claims, which comprises:

(i) preparing the oil-in-water microemulsion by mixing the oily phase B) with the continuous aqueous phase, said oily phase B) containing ozonized oil, preferably ozonized plant oil;
(ii) encapsulating the oil-in-water microemulsion with a coating comprising a cationic surfactant containing quaternary ammonium groups, a polysaccharide and a polysaccharide modified with quaternized ammonium groups;
(iii) dispersing the microstructures thus obtained in the aqueous phase A).

12. Composition according to any one of the claims from 1 to 10, for use in the treatment of cutaneous lesions, such as ulcers, erosions, abrasions, scars and the like.

13. Composition according to any one of the claims from 1 to 10, in the form of suppository for rectal administration.

14. Use of a composition according to any one of the claims from 1 to 10 for the cosmetic treatment of the skin.

15. Textile material treated with a composition according to any one of the claims from 1 to 10.

**Patentansprüche**

1. Zusammensetzung, enthaltend Mikrostrukturen, die in einer wässrigen Phase A) dispergiert sind, wobei die Mikrostrukturen Folgendes enthalten:

eine interne Phase, bestehend aus einer Öl-in-Wasser-Mikroemulsion (o/w), enthaltend eine ölige Phase B), die in einer kontinuierlichen wässrigen Phase dispergiert ist, wobei die ölige Phase B) ein ozonisiertes Öl enthält;

eine Beschichtung der internen Phase, enthaltend ein kationisches Tensid, enthaltend mindestens eine quaternäre Ammoniumgruppe, ein Polysaccharid und ein mit quaternierten Ammoniumgruppen modifiziertes Polysaccharid.

2. Zusammensetzung nach Anspruch 1, wobei das ozonisierte Öl ein ozonisiertes Pflanzenöl ist, das vorzugsweise aus Folgenden ausgewählt ist: Sonnenblumenöl, Olivenöl, Erdnussöl, Arganöl, Traubenkernöl, Jojobaöl, Sojabohnenöl, Maisöl, Palmöl, Baumwollsaatöl, Rapsöl, Kokosöl, Rizinusöl, Leinsamenöl, Borretschöl, Nachtkerzenöl oder Gemischen daraus.

3. Zusammensetzung nach einem der vorstehenden Ansprüche, wobei die kontinuierliche wässrige Phase Folgendes enthält:

Wasser: 50-90 %;
nicht-ionisches Tensid: 10-50 %;
wobei die Prozentangaben Gewichtprozent sind, bezogen auf das Gesamtgewicht der kontinuierlichen wässrigen Phase.

4. Zusammensetzung nach einem der vorstehenden Ansprüche, wobei das kationische Tensid mindestens eine quaternäre Ammoniumgruppe der folgenden Formel enthält:

$$X^-N^+R_1R_2R_3R_4$$

wobei $R_1$ ein $C_8$-$C_{20}$-Alkyl, vorzugsweise $C_{10}$-$C_{15}$, unverzweigt oder verzweigt, ist;
$R_2$, $R_3$, $R_4$ gleich oder verschieden, $C_1$-$C_4$-Alkyle, unverzweigt oder verzweigt, vorzugsweise Methyle sind;
$X^-$ ein Gegenion, vorzugsweise ein Halogenidion, noch bevorzugter Chlorid oder Bromid ist.

5. Zusammensetzung nach einem der vorstehenden Ansprüche, wobei das Polysaccharid, das sich von dem mit quaternierten Ammoniumgruppen modifizierten Polysaccharid unterscheidet, aus Folgenden ausgewählt ist: Cellulose, Carboxymethylcellulose, Hydroxyethylcellulose, Methylcellulose, Hydroxypropylethylcellulose, Xanthan-Gummi, Guar-Gummi, Pektinen, Arabinogalactanen, Galactomannanen, Carboxymethylgalactomannanen, Albumin, Alginaten oder Gemischen daraus.

6. Zusammensetzung nach einem der vorstehenden Ansprüche, wobei das mit quaternierten Ammoniumgruppen modifizierte Polysaccharid aus Folgenden ausgewählt ist: Guarhydroxypropyltriamoniumchlorid, quaternierter Hydroxymethylcellulose oder Gemischen daraus.

7. Zusammensetzung nach einem der vorstehenden Ansprüche, wobei die Beschichtung Folgendes enthält:

kationisches Tensid: 5 % - 30 %;
Polysaccharid: 10 % - 40 %;
Polysaccharid, modifiziert mit 40 % - 80 %;
quaternierten Ammoniumgruppen;
wobei die Prozentangaben Gewichtprozent sind, bezogen auf das Gesamtgewicht der Beschichtung.

8. Zusammensetzung nach einem der vorstehenden Ansprüche, wobei das ozonisierte Öl in einer Menge von 0,1 Gew.-% bis 25 Gew.-%, vorzugsweise von 1 Gew.-% bis 10 Gew.-%, bezogen auf das Gesamtgewicht der Zusammensetzung, vorhanden ist.

9. Zusammensetzung nach einem der vorstehenden Ansprüche, wobei die ölige Phase B) und die kontinuierliche wässrige Phase in einem Gewichtsverhältnis von 5:95 bis 40:60 vorhanden sind.

10. Zusammensetzung nach einem der vorstehenden Ansprüche, die die folgende Zusammensetzung aufweist:

wässrige Phase A) 20-70 Gew.-%;
interne Phase 5-50 Gew.-%;
Beschichtung 0,1-40 Gew.-%;
bezogen auf das Gesamtgewicht der Zusammensetzung.

**11.** Verfahren zur Herstellung einer Zusammensetzung nach einem der vorstehenden Ansprüche, das Folgendes enthält:

(i) Herstellen der Öl-in-Wasser-Mikroemulsion durch Vermischen der öligen Phase B) mit der kontinuierlichen wässrigen Phase, wobei die ölige Phase B) ozonisiertes Öl, vorzugsweise ozonisiertes Pflanzenöl, enthält;
(ii) Einkapseln der Öl-in-Wasser-Mikroemulsion mit einer Beschichtung, enthaltend ein kationisches Tensid, enthaltend quaternäre Ammoniumgruppen, ein Polysaccharid und ein mit quaternierten Ammoniumgruppen modifiziertes Polysaccharid;
(iii) Dispergieren der so erhaltenen Mikrostrukturen in der wässrigen Phase A).

**12.** Zusammensetzung nach einem der Ansprüche 1 bis 10 zur Verwendung bei der Behandlung von Hautläsionen wie Geschwüren, Erosionen, Abschürfungen, Narben und dergleichen.

**13.** Zusammensetzung nach einem der Ansprüche 1 bis 10 in Form eines Zäpfchens zur rektalen Verabreichung.

**14.** Verwendung einer Zusammensetzung nach einem der Ansprüche 1 bis 10 zur kosmetischen Behandlung der Haut.

**15.** Textilmaterial, das mit einer Zusammensetzung nach einem der Ansprüche 1 bis 10 behandelt wurde.

## Revendications

**1.** Composition comprenant des microstructures dispersées dans une phase aqueuse A), dans laquelle lesdites microstructures comprennent :

une phase interne constituée d'une microémulsion huile dans l'eau (o/w) comprenant une phase huileuse B) dispersée dans une phase aqueuse continue, ladite phase huileuse B) comprenant une huile ozonisée ;
un revêtement de ladite phase interne comprenant un tensioactif cationique contenant au moins un groupe ammonium quaternaire, un polysaccharide et un polysaccharide modifié par des groupes ammonium quaternisés.

**2.** Composition selon la revendication 1, dans laquelle l'huile ozonisée est une huile végétale ozonisée, préférablement sélectionnée parmi : huile de tournesol, huile d'olive, huile d'arachide, huile d'argan, huile de pépins de raisin, huile de jojoba, huile de soja, huile de maïs, huile de palme, huile de coton, huile de colza, huile de coco, huile de ricin, huile de lin, huile de bourrache, huile d'onagre, ou leurs mélanges.

**3.** Composition selon l'une quelconque des revendications précédentes, dans laquelle la phase aqueuse continue comprend :

eau : de 50 % à 90 % ;
tensioactif non ionique : de 10 % à 50 % ;
dans laquelle les pourcentages sont des pourcentages en poids par rapport au poids total de la phase aqueuse continue.

**4.** Composition selon l'une quelconque des revendications précédentes, dans laquelle ledit tensioactif cationique contient au moins un groupe ammonium quaternaire ayant la formule :

$$X^-N^+R_1R_2R_3R_4$$

dans laquelle $R_1$ est un alkyle en $C_8$-$C_{20}$, préférablement en $C_{10}$-$C_{16}$, linéaire ou ramifié ;
$R_2$, $R_3$, $R_4$ sont identiques ou différents les uns des autres, des alkyles en $C_1$-$C_4$, des alkyles linéaires ou ramifiés, préférablement des méthyles ;
$X^-$ est un contre-ion, préférablement un ion halogénure, plus préférablement chlorure ou bromure.

**5.** Composition selon l'une quelconque des revendications précédentes, dans laquelle ledit polysaccharide, différent du polysaccharide modifié par des groupes ammonium quaternisés, est sélectionné parmi : cellulose, carboxyméthylcellulose, hydroxyéthylcellulose, méthylcellulose, hydroxypropyléthylcellulose, gomme de xanthane, gomme de guar, pectines, arabinogalactanes, galactomannanes, carboxyméthylgalactomannanes, albumine, alginates, ou leurs mélanges.

6. Composition selon l'une quelconque des revendications précédentes, dans laquelle ledit polysaccharide modifié par des groupes ammonium quaternisés, est sélectionné parmi : chlorure d'hydroxypropyltriamonium de guar, hydroxyméthylcellulose quaternisée, ou leur mélange.

7. Composition selon l'une quelconque des revendications précédentes, dans laquelle ladite phase aqueuse continue comprend :

   tensioactif cationique : de 5 % à 30 % ;
   polysaccharide : de 10 % à 40 % ;
   polysaccharide modifié avec de 40 % à 80 % de groupes ammonium quaternisés ;
   dans laquelle les pourcentages sont des pourcentages en poids par rapport au poids total du revêtement.

8. Composition selon l'une quelconque des revendications précédentes, dans laquelle l'huile ozonisée est présente en une quantité de 0,1 % à 25 % en poids, préférablement de 1 % à 10 % en poids, par rapport au poids total de la composition.

9. Composition selon l'une quelconque des revendications précédentes, dans laquelle la phase huileuse B) et la phase aqueuse continue sont présentes dans un rapport pondéral allant de 5:95 à 40:60.

10. Composition selon l'une quelconque des revendications précédentes ayant la composition suivante :

    phase aqueuse A) de 20 % à 70 % en poids ;
    phase interne de 5 % à 50 % en poids ;
    revêtement de 0,1 % à 40 % en poids ;
    par rapport au poids total de la composition.

11. Procédé pour préparer une composition selon l'une quelconque des revendications précédentes, qui comprend :

    (i) la préparation de la microémulsion huile dans l'eau par mélange de la phase huileuse B) avec la phase aqueuse continue, ladite phase huileuse B) contenant de l'huile ozonisée, de préférence de l'huile végétale ozonisée ;
    (ii) l'encapsulage de la microémulsion huile dans l'eau avec un revêtement comprenant un tensioactif cationique contenant des groupes ammonium quaternaire, un polysaccharide et un polysaccharide modifié par des groupes ammonium quaternisés ;
    (iii) la dispersion des microstructures ainsi obtenues dans la phase aqueuse A).

12. Composition selon l'une quelconque des revendications 1 à 10, pour une utilisation dans le traitement de lésions cutanées, telles que des ulcères, des érosions, des abrasions, des cicatrices et analogue.

13. Composition selon l'une quelconque des revendications 1 à 10, sous forme de suppositoire pour administration rectale.

14. Utilisation d'une composition selon l'une quelconque des revendications 1 à 10 pour le traitement cosmétique de la peau.

15. Matériau textile traité avec une composition selon l'une quelconque des revendications 1 à 10.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012038061 A